Europäisches Patentamt

(19) European Patent Office  (11) Publication number: **0 056 697**

Office européen des brevets  **B1**

(12) **EUROPEAN PATENT SPECIFICATION**

(45) Date of publication of patent specification: **08.07.87**  (51) Int. Cl.⁴: **A 61 N 1/40,** A 61 K 9/50

(21) Application number: **82300125.0**

(22) Date of filing: **11.01.82**

(54) **Injectable compositions suitable for use in inductively heating neoplasms.**

(30) Priority: **09.01.81 US 223727**

(43) Date of publication of application:
**28.07.82 Bulletin 82/30**

(45) Publication of the grant of the patent:
**08.07.87 Bulletin 87/28**

(84) Designated Contracting States:
**AT BE CH DE FR GB IT LI LU NL SE**

(56) References cited:
**EP-A-0 040 512**
**DE-A-1 284 528**
**DE-C- 523 823**
**FR-A- 750 400**
**FR-A-1 244 347**
**FR-A-2 160 410**
**FR-A-2 291 773**
**LU-A- 71 825**
**US-A-1 873 808**
**US-A-3 279 996**
**US-A-3 543 762**
**US-A-3 638 657**
**US-A-4 106 488**

(73) Proprietor: **Rand, Robert W.**
**521 N. Bristol Street**
**Los Angeles California 90049 (US)**
(73) Proprietor: **Snow, Harold D.**
**4201 Noble Avenue**
**Sherman Oaks California 91403 (US)**
(73) Proprietor: **Elliott, David G.**
**737 W. Starlight Heights**
**La Canada California 91011 (US)**
(73) Proprietor: **Haskins, Glen M.**
**3811 Rio Hondo Avenue**
**Rosemead California 91770 (US)**

(72) Inventor: **Rand, Robert W.**
**521 N. Bristol Street**
**Los Angeles California 90049 (US)**
Inventor: **Snow, Harold D.**
**4201 Noble Avenue**
**Sherman Oaks California 91403 (US)**
Inventor: **Elliott, David G.**
**737 W. Starlight Heights**
**La Canada California 91011 (US)**
Inventor: **Haskins, Glen M.**
**3811 Rio Hondo Avenue**
**Rosemead California 91770 (US)**

(74) Representative: **Spencer, Graham Easdale et al**
**A.A. Thornton & CO Northumberland House**
**303-306, High Holborn**
**London WC1V 7LE (GB)**

Courier Press, Leamington Spa, England.

## Description

The present invention is concerned with injectable compositions for use in causing hyperthermia of neoplasms and consequent necrosis thereof.

It is known to treat aberrant cells in animal bodies (neoplasms) by hyperthermia; this causes necrosis of the neoplasms because the latter are more susceptible to heat than normal cells. Various methods of heating have been proposed for such treatment, for example, heating by means of radio-frequency or microwave radiation, and inductive heating using an alternative magnetic field.

A radio frequency or microwave field only causes heating at or adjacent to the surface of a body because of the tendency of the body to absorb radio frequency electromagnetic radiation (the depth to which such radiation penetrates the body depends upon the frequency of the radiation). Such body tissue heating as is caused by radio frequency radiation has two principal components: eddy current heating and di-electric heating. Heating of this type can be achieved in the absence of any foreign material in the tissue being heated.

Induction heating of body tissue as a result of the presence of a material having hysteresis characteristics (referred to as hysteresis heating) involves the application of a magnetic field which is generally unattenuated by tissue. Hence, induction heating can reach any desired depth or level beneath the surface of the tissue. In order to achieve this manner of heating in tissue, it is necessary to locate a material exhibiting magnetic hysteresis close to the site where the heat is required.

Induction-hysteresis heating is generally accompanied by some eddy current and dielectric heating, but the amount of eddy current and dielectric heating can be minimized by using a comparatively low frequency alternating magnetic field. The amount of such heating varies directly with the square of the frequency of the field, while the amount of hysteresis heating varies directly with the frequency. Thus, in general, the lower the frequency of the field the less undesired eddy current heating relative to the amount of the desired hysteresis heating. (The frequency, of course, should be sufficiently high to ensure the desired degree of hysteresis heating).

The limited amount that a radio frequency field can normally penetrate body tissue is an important factor limiting the use of this type of heating in the treatment of neoplasms since radio frequency heating can not be utilized effectively in causing hyperthermia in aberrant growths which are so far beneath the surface of tissue that they are not reached by the radio frequency field. The use of such a field to cause hyperthermia at or adjacent to the surface of the skin or at or adjacent to the interior of an incision within the body is limited by another important factor — the problem of selectively concentrating the heat so that neoplasm is heated sufficiently to cause necrosis without related or corresponding heating of adjacent tissue.

This can be illustrated by referring to specific temperatures which have previously been recognized to be important in causing necrosis by hyperthermia. It is believed that holding the body tissue temperature at 40°C generally will not cause necrosis, but a temperature of 42°C will, if the body tissue is maintained at this temperature for long enough, while a temperature of 60°C or more causes practically instantaneous necrosis. Body tissue should not, of course; be subjected to temperatures near to 100°C for even a very short time because of the danger of vaporisation of water; in practice, therefore, the tissue is not normally heated to more than 90°C.

The time necessary to cause necrosis at a given temperature is not directly dependent on the temperature in the usual manner of temperature-dependent reactions; in addition, certain parts of the body or neoplasms are more susceptible, to necrosis at an elevated temperature than other parts of the body and/or other neoplasms. Because of these factors, it is impossible to give exact relationships between the time and the temperature necessary to accomplish necrosis of neoplasms. Another factor which is important in considering the amount of hyperthermia to cause necrosis in neoplasm is the tendency of the body and/or a particular portion of the body to serve as a heat insulator which tends to concentrate heat developed, for example, at a specific source.

With radio frequency or microwave hyperthermia, the difficulty in confining the field necessary to cause necrosis of a specific area or region will normally cause necrosis of healthy tissue in an adjacent area or region. While to a degree this can be counteracted by the use of special electrodes, it is impossible to adequately control radio frequency or microwave heating in many applications so as to avoid damage to adjacent normal cells or tissue. While it may be possible to improve the concentration of the heating effects achieved with radio frequency or microwave heating by the implantation of a metallic conductor or similar material in a specific area where concentrated heating is desired, the use of a conductor is not always advantageous because it does not improve the depth of penetration of tissue by the radio frequency field.

The use of induction heating in causing necrosis of neoplasm alleviates several problems involved in radio frequency hyperthermia treatment. When induction heating is used for this purpose, it is necessary to utilize an invasive technique to locate a material having a magnetic permeability greater than unity and capable of exhibiting hysteresis losses, for example, ferromagnetic particles such as a conventional ferrite or conventional iron or steel powders, in or immediately adjacent to the neoplastic growth. The application of an alternating magnetic field results in hysteresis heating within the magnetic material itself, which avoids the problem of containing or limiting the heated area obtained which arises when using a radio frequency field. Because of the penetration of low frequency magnetic fields into body tissue, it is possible to use a magnetic material well beneath the surface of the body.

Prior proposals for induction heating to cause necrosis suffer from several disadvantages. In particular, difficulties have been experienced in effectively immobilising the implanted magnetic material either within or immediately adjacent to the neoplasm so as to be able to provide localised heating without danger of the magnetic material moving from its initial location within the body.

U.S. Patent 4,106,488 describes a process in which a composition containing magnetic particles is injected intravenously and the particles are preferentially absorbed by cancerous cells to provide a local concentration of particles in the neoplasm which are then inductively heated to cause hyperthermia.

According to the present invention, there is provided an injectable composition for use in causing hyperthermia of neoplasms and consequently necrosis thereof, which composition comprises particles having a magnetic permeability greater than one and a pharmacologically acceptable carrier liquid, characterised in that the carrier liquid is curable in living tissue.

In a preferred embodiment of the invention, the magnetic particles have thereon an adherent protective coating of pharmacologically acceptable material.

The composition of the invention may be used to treat neoplasms in a wide variety of locations by injecting into tissue close to the site of the neoplasms and subjecting the latter site to an alternating current so as to cause hysteresis heating of the particles.

The composition should be liquid so that it can be injected via a conventional hypodermic needle or by a similar method, such as, trans-arterial catherization.

The magnetic particles may be of any magnetic material having a magnetic permeability of greater than unity and thereby exhibiting hysteresis heating. As a practical matter, it is preferred to use magnetic materials which are pharmacologically acceptable (non-toxic) and which have as large a hysteresis loop when subjected to an alternating field as possible. (It is the area within the hysteresis loop which is of prime importance since this area dictates the efficiency of the hysteresis heating; the exact shape of the hysteresis loop is relatively unimportant).

The magnetic particles are preferably ferromagnetic; preferred are conventional ferrites and various commercially obtainable iron and steel particles known to have comparatively large magnetic losses. Such materials are relatively inert as far as most metabolic processes are concerned and may be used in most parts of the body without significant concern. Other biologically inert particles which exhibit hysteresis heating may, of course, be employed.

The maximum temperature achieved during induction heating can be controlled by the selection of magnetic particles having an appropriate Curie point. Thus, for example, it is possible to choose a particular paramagnetic or ferromagnetic composition having a Curie point anywhere within the range of from 42°C to 90°C so that hyperthermia of a neoplasm may be accomplished without heating tissue beyond the desired temperature. (Normally the magnetic characteristics of a material will change over a comparatively small range of temperatures and therefore the Curie point corresponds to a limited range of temperatures).

The particles utilized are preferably sufficiently small that they are capable of forming either a slurry or colloidal suspension in the carrier fluid. The slurry should have a low viscosity comparable to that of a suspension of aluminium hydroxide antacid in water and be capable of being easily handled and poured from a bottle to a spoon. As a practical matter, it is preferred that the particles used have an average diameter of no greater than one millimetre (although larger particles may be used).

The magnetic particles should not be too fine, because this tends to make the hysteresis loop exhibited by the material too small; the particles should be of such dimension that there is no diminution of the hysteresis loop. With at least some materials, comparatively long, thin particles have better hysteresis heating characteristics than particles of an approximately spherical shape.

As a practical matter, the lower size limit of the particles and the shape of the particles should be determined on an empirical basis by testing particles of various sizes, in general, however, none of the particles should have a dimension less than the dimensions of the magnetic domains of the material in the particles. In general, it is undesirable to use particles any finer than those which are conventionally used in ferro-fluids in which ferromagnetic particles are in colloidal suspension.

Virtually any type of biologically inert liquid which is easily tolerated by the body and which is curable in living tissue may be used as the carrier liquid. The advantage of using a curable carrier liquid in the composition of the present invention is that the composition may be used in any location, including blood vessels, with no risk of the particles moving within the body once the carrier liquid is cured.

Minor amounts of biologically inert surfactants and the like may be employed in the composition to facilitate the suspension of the particles used and to aid in controlling the viscosity of the mixture.

As indicated, the magnetic particles used are preferably completely coated with a biologically inert, adherent, conventional protective layer in order to minimize interference with normal metabolic processes. Many different coatings may be used, but coatings based on polyolefins are particularly preferred. The coatings used on the particles should, of course, be as thin as reasonably possible; a small amount of agglomeration of the particles caused by the presence of the coating material is not generally detrimental.

According to the present invention, the carrier liquid is curable in body tissue, preferably as the result of a polymerization or similar action initiated by one or more components present in the carrier liquid or as a result of the application of external energy. This energy may be derived from normal body heat or directly from the magnetic field used in causing the hysteresis heating, or it may be the heat resulting from such heating of the particles within the carrier liquid.

As indicated, the primary reason why a curable carrier liquid is used in the composition of the invention is the desirability of creating a physical structure within the tissue which will physically hold the magnetic particles so that they cannot move throughout the body in the blood and possibly become lodged in various passages or cells where they might cause damage. The precise method of curing the composition of the invention to form a solid or a non-Newtonian semi-solid is in many respects immaterial to the present invention.

Further, the solidity of the final cured non-liquid body or composition is also a matter of choice, but it is preferably of a somewhat flexible, somewhat elastomeric character, corresponding to the character of human tissue or it should be of non-Newtonian character capable of being deformed while still remaining as a coherent unit. It is particularly preferable to use as the carrier liquid a material which is curable to an elastomer within the body.

The carrier liquid may contain a wide variety of biologically inert secondary ingredients such as fumed silica, surfactants, and the like, serving various secondary functions such as, for example, a degree of control of the ultimate elasticity of the polymer body produced. The carrier liquid employed may be a prepolymer or partially polymerized solution containing one or more catalysts, and may further contain an inert liquid such as water, which serves merely as a diluent viscosity control agent until such time as the carrier liquid is cured.

It is presently preferred to utilize as the carrier liquid a silicone elastomer composition which is capable of being injected into the body as a liquid and which will cure when in place within the body to physically retain the magnetic particles in the intended location within the body. The use of this material is particularly preferred because mixtures of this type of material with the particles of the invention are normally adequately coherent and they do not separate or break up into smaller units. Further, silicone elastomers are normally acceptable for use within the human body.

It is desirable that the curable carrier liquid should wet the magnetic particles so that they are completely coated by the carrier liquid in such a way that they are effectively isolated from the normal body fluids; this minimizes any chance of the material within the particles interfering with normal metabolic processes. It also makes it possible to use particles of a magnetic material which is somewhat biologically active. The use of such a biologically active material is not, however, preferred because of the possibility of some sort of unintended interference with normal metabolic processes.

The preferred relative proportions of particles and carrier liquid in the composition of the invention will depend upon the physical properties of the components used. In general, the mixture should contain the minimum amount of carrier liquid which is effective to suspend and hold the particles so that they do not separate out of the mixture during the time that the mixture is being injected into the body. Further, the proportions should be such that enough carrier liquid is present that the cohesive character of the liquid tends to hold the mixture together as a unit after the mixture is injected into the body.

This is particularly important, for example, when the composition is injected into a blood vessel. The mixture should not fragmentize in the blood vessel, but should hold together by cohesion until it is cured. In certain applications, it is highly desirable for the mixture of particles and carrier liquid to be sufficiently coherent that the composition may be guided to a specific location within or adjacent to neoplasm by the use of an external magnetic field, until such time as the composition is rendered sufficiently non-liquid that it is held in place as a result of physical engagement with adjacent walls or tissue.

In some cases, to increase the amount of hysteresis heating attainable at a given magnetic field intensity, it may be desirable to apply a DC magnetic field to the composition while it is solidifying. This serves to align all of the magnetic particles in one direction. Subsequent applications of the AC magnetic field along the same axis will cause more magnetic heating than if the particles were randomly oriented.

The composition according to the invention may contain temperature-indicating ingredients which can be monitored by X-ray, ultrasonic, or other means while the induction heating is in progress.

The composition according to the invention is located by injection or similar means close to a neoplasm and cured; the tissue containing the neoplastic growth is then subjected to an alternating magnetic field, as discussed above, which causes sufficient hyperthermia to cause necrosis of the neoplasm adjacent to the magnetic particles of the composition. Heating may be produced using any sort of coil which is suitable for induction heating purposes.

**Claims**

1. An injectable composition for use in causing hyperthermia of neoplasms and consequent necrosis thereof, which composition comprises particles having a magnetic permeability greater than one and a pharmacologically acceptable carrier liquid, characterised in that the carrier liquid is curable in living tissue.

2. A composition according to claim 1, in which the magnetic particles have thereon an adherent protective coating of a pharmacologically acceptable material.

3. A composition according to claim 1 or 2, in which the carrier liquid comprises a catalytically polymerisable material and a polymerisation catalyst therefor.

4. A composition according to claim 3, in which the polymerisable material is a silicone which forms an elastomer on polymerisation.

5. A composition according to any of claims 1 to 4, in which it is sufficiently cohesive to remain together after injection into living tissue.

6. A composition according to any of claims 1 to 5, in which carrier liquid is present in an amount sufficient to wet all the surfaces of the magnetic particles.

7. A composition according to any of claims 1 to 6, in which none of the magnetic particles have a dimension less than the dimensions of the magnetic domains of the material.

8. A composition according to any of claims 1 to 7, in which the magnetic particles are of a ferrite material.

9. A composition according to any of claims 1 to 8, in which the magnetic particles have a Curie point of less than 90°C.

## Patentansprüche

1. Eine injizierbare Zusammensetzung für die Verwendung beim Verursachen von Hyperthermie von Neoplasmen und deren darauffolgende Nekrose, wobei die Zusammensetzung Partikel enthält, die eine magnetische Permeabilität aufweisen, die größer als 1 ist sowie eine pharmakologisch annehmbare Trägerflüssigkeit, dadurch gekennzeichnet, daß die Trägerflüssigkeit in lebendem Gewebe zum Gerinnen gebracht werden kann.

2. Eine Zusammensetzung nach Anspruch 1, in der die magnetischen Partikel einen anhaftenden, schützenden Überzug eines pharmazeutisch annehmbaren Materials um sich herum aufweisen.

3. Eine Zusammensetzung nach Anspruch 1 oder 2, in der die Trägerflüssigkeit ein katalytisch polymerisierbares Material sowie einen Polymerisationskatalysator für dieses enthält.

4. Eine Zusammensetzung nach Anspruch 3, in der das polymerisierbare Material ein Silicon ist, das nach Polymerisierung ein Elastomer bildet.

5. Eine Zusammensetzung nach einem der Ansprüche 1 bis 4, die ausreichend cohäsiv ist, um nach Injektion in lebendes Gewebe zusammenzubleiben.

6. Eine Zusammensetzung nach einem der Ansprüche 1 bis 5, in der die Trägerflüssigkeit in einer ausreichenden Menge vorliegt, um sämtliche Oberflächen der magnetischen Partikel anzufeuchten.

7. Eine Zusammensetzung nach einem der Ansprüche 1 bis 6, in der keines der magnetischen Teilchen eine Größenordnung aufweist, die geringer ist als die Größenordnung der magnetischen Domänen des Materials.

8. Eine Zusammensetzung nach einem der Ansprüche 1 bis 7, in der die magnetischen Teilchen aus Ferrit-Material sind.

9. Eine Zusammensetzung nach einem der Ansprüche 1 bis 8, in der die magnetischen Teilchen einen Curiepunkt von weniger als 90°C aufweisen.

## Revendications

1. Une composition injectable à utiliser pour provoquer l'hyperthermie des néoplasmes et leur nécrose qui en résulte, cette composition comprenant des particules ayant une perméabilité magnétique supérieure à 1 et un véhicule liquide acceptable en pharmacie, caractérisée en ce que le véhicule liquide est durcissable dans le tissu vivant.

2. Une composition selon la revendication 1, dans laquelle les particules magnétiques portent un revêtement protecteur adhérent d'une substance acceptable en pharmacie.

3. Une composition selon la revendication 1 ou 2, dans laquelle le véhicule liquide consiste en une matière catalytiquement polymérisable et un catalyseur de polymérisation à cet effet.

4. Une composition selon la revendication 3, dans laquelle la matière polymérisable est une silicone qui forme un élastomère par polymérisation.

5. Une composition selon l'une quelconque des revendications 1 à 4, dans laquelle il est suffisamment cohérent pour rester ensemble après l'injection dans le tissu vivant.

6. Une composition selon l'une quelconque des revendications 1 à 5, dans laquelle le véhicule liquide est présent en quantité suffisante pour mouiller les surfaces de toutes les particules magnétiques.

7. Une composition selon l'une quelconque des revendications 1 à 6, dans laquelle aucune des particules magnétiques n'a une dimension inférieure aux dimensions des domaines magnétiques de la matière.

8. Une composition selon l'une quelconque des revendications 1 à 7, dans laquelle les particules magnétiques consistent en une matière ferritique.

9. Une composition selon l'une quelconque des revendications 1 à 8, dans laquelle les particules magnétiques ont un point de Curie de moins de 90°C.